# EUROPEAN PATENT SPECIFICATION

(11) **EP 0 567 202 B1**
(45) Date of publication and mention of the grant of the patent: **10.06.1998**
(21) Application number: 93202119.9
(22) Date of filing: 22.10.1990
(51) Int. Cl.: A61M 39/00, A61M 5/162

(54) **Injection site**
Injektionsstelle
Site d'injection

(30) Priority: 23.10.1989 US 425790
(43) Date of publication of application: 27.10.1993
(62) Divisional of application: 91900074.5
(73) Proprietor: BAXTER INTERNATIONAL INC., Deerfield, Illinois 60015 (US)
(72) Inventor: Dudar, Thomas E., Palatine, Illinois 60067 (US); Jepson, Steven C., Palatine, Illinois 60067 (US); Dobbie, Robert P., Lincolnshire, Illinois 60069 (US); Graham, Peter L., Gurnee, Illinois 60031 (US); Finley, Michael J., Park City, Illinois 60085 (US); Rollins, Richard A., Mundelein, Illinois 60060 (US)
(74) Representative: MacGregor, Gordon

(56) References cited:
- EP-A- 0 319 764
- WO-A-89/06553
- US-A- 4 559 043

## Description

The invention pertains to an injection site usable with a blunt cannula in the medical field for handling medications and body fluids.

In the medical field, injection sites usable with pointed cannulae have long been known. For example, such sites can be formed with a housing having a fluid flow path therein. A septum is positioned in the housing closing the fluid flow path.

Injection sites usable with a blunt cannula are known from US-A-4197848 and WO-A-89/06553.

WO-A-89/06553 discloses an injection site including a housing which defines a fluid flow channel therethrough. The housing has a first and a second end.

A flexible sealing member is carried by the housing for sealing the first end. The sealing member has a resealable opening therein. The sealing member also is formed with a curved exterior peripheral surface such that the blunt cannula can be sealingly inserted through the opening and placed in fluid flow communication with the flow path. Further, the blunt cannula can be removed from the opening with the sealing member then interacting with the housing so as to reseal the opening.

The housing can also be formed with the first end including an annular channel underlying the sealing member. The sealing member is subjected to radially directed forces by a tapered surface of the first end of the housing. These forces tend to reseal the opening in the sealing member.

The sealing member can be a cylindrically shaped rubber member. The first end of the housing can include an interior tapered surface for receiving the sealing member and for applying the radially directed forces to the sealing member.

A retaining member carried by the first end of the housing can be used to retain the sealing member with the housing. The retaining member can be generally U-shaped. Alternately, the retaining member can he formed as a coiled spring.

The retaining member applies axially directed forces to the sealing member. In one construction, the retaining member deflects the sealing member and forms a curved exterior peripheral surface thereon. The curved exterior peripheral surface is an easily wipeable surface.

The retaining member deflects or distorts the upper and lower peripheral edges slightly as a result of applying axial forces thereto. When the blunt cannula is inserted into the slit in the sealing member, an annular interior peripheral region of the sealing member deforms further and fills, at least in part, the annular channel.

Deformation of this annular peripheral region results in an insertion force in a range of 900 to 2300 g. (2.0 to 5 pounds). Preferably, the insertion force will have a value of the order of 900 g. (2.0 pounds).

The resealable opening in the sealing member can extend entirely through that member. Alternately, the resealable opening can extend only partway therethrough and the end of the blunt cannula will be used to tear through the remainder of the sealing member.

The sealing member can be formed in two parts. An exterior cylindrical portion can be slit completely. An interior cylindrical unslit portion can be provided to seal the site until the blunt cannula is inserted therethrough the first time.

The interior surface of the first end can be formed with the taper in a range on the order of 5 degrees to 20 degrees. Preferably, the interior surface will have a taper on the order of 12 degrees. This tapered surface permits the use of a cylindrically shaped sealing member.

To provide for leak-free insertion, the length of the slit in the sealing member must be less than one-half of the circumference of the cannula being inserted therethrough. Hence, the slit length may exceed the diameter of the cannula being inserted. In addition, the slit length must be great enough, given the elastic limit of the sealing member, to prevent tearing during insertion.

The present invention concerns an injection site which may be made as set out above, but is adapted for coupling to standard vials.

The precharacterising part of Claim 1 is based on the disclosure of WO-A-89/06553 and the characterising part sets out the distinguishing features of the invention.
Fig 1 shows a cross-sectional view of a pre-slit injection site (which is not according to the invention) with a vial adapter lockingly engaged to a vial; and
Fig 2 shows a cross-sectional view of a preslit injection site according to the present invention.

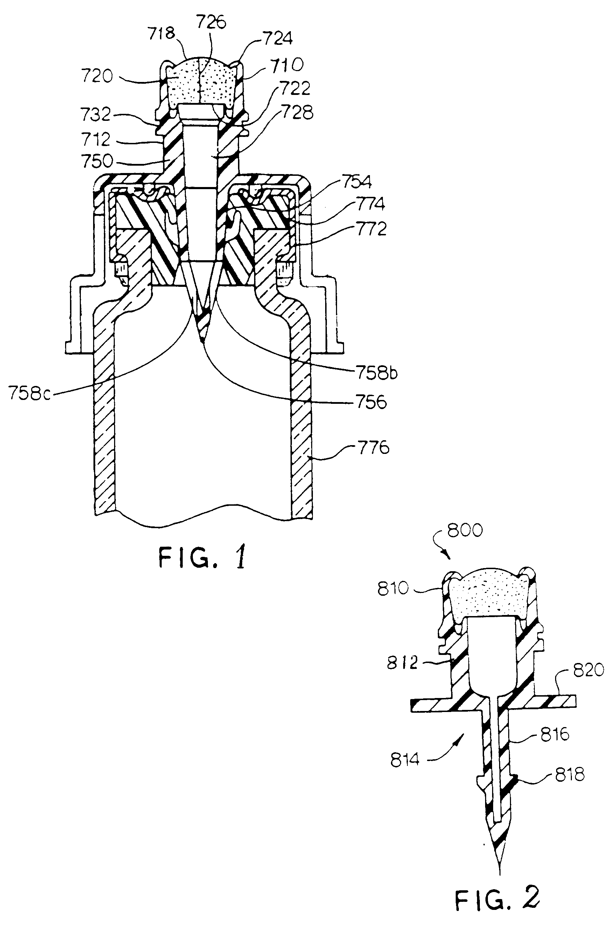

Reference is made to WO-A-89/06533, Figs 2 to 28, and the accompanying description. This disclosure provides a full enabling disclosure for making the injection sites disclosed below.

Fig 1 discloses an injection site, which is not in accordance with the present invention. The injection site is of the type disclosed in WO-A-89/06553 in combination with a coupling component to permit the repeated access to a standard drug vial without using sharp needles. A pre-slit injection site 710 is formed with a cylindrical housing 712 having a first end 714 and a second end 716. A resealable septum 720 is carried by the first end 714 and has inner surface 722 and an outer surface 718, which has been forced into a dome-like shape by annular, U-shaped, swaged end members 724. The septum has a preformed opening 726. The housing 712 carries a hollow fluid flow member 728 connecting the first and second ends 714,716.

The fluid flow member 728 communicates with an adapter spike 750 having a generally cylindrical, hollow body portion 754, which tapers into a solid centre point 756. Located in centre point 756 are peripheral openings 758b,758c, so that a continuous flow passageway is established from the injection site 710 through the adapter spike 750.

In use, a standard drug vial 776 having a vial closure 772 of aluminium carrying a rubber stopper 774 is prepared. The adapter spike 750 is positioned at the centre of the rubber stopper 774 and pressed firmly down towards vial 776 so that the centre point 756 pierces through the stopper. To reduce the insertion force, required, the adapter spike 750 can be lubricated, for example, with silicone, prior to insertion of the adapter into the stopper.

In a modification according to the present invention, a device 800, shown in Fig 2, comprises an injection site 810 molded with a spike connector 814. The body 816 of the spike connector is provided with a barb 818. As with adapter spike 750, described above, the spike connector can be inserted through a stopper of a drug vial. Upon complete insertion, the connector wall 820 between the injection site housing 812 and the spike body 816 come to rest on top of the vial closure. The barb 818 is inserted through the stopper and provides resistance against disengagement of the device 800 from the vial.

## Claims

1. An injection site (800) usable with a blunt cannula and comprising a housing (812) defining a fluid flow channel therethrough, resilient sealing means, carried by said housing (812) overlying said channel for sealing a first end of the channel, the sealing means being formed with a resealable opening therein extending at least partway therethrough and having an exterior surface; retaining means for retaining said sealing means in said housing such that a blunt cannula can be sealingly inserted through said opening and placed in fluid flow communication with said flow channel and such that the cannula can be removed therefrom with said sealing means interacting with said retaining means so as to reseal said opening, said retaining means including a deformation of said housing first end against said exterior surface of said sealing means, said first end deformation applying axially directed forces to said sealing means, and a coupling component (814) integral with said second end and continuing said fluid flow channel, said coupling component having a spike having a generally cylindrical, hollow body portion (816);
characterised in that the body portion (816) of the spike tapers to a solid centre point to permit piercing of the stopper of a drug vial, the solid centre point having an opening to provide drainage of fluid from the vial through the fluid flow channel, the body portion (816) including a barb member (818) to provide resistance against disengagement of the injection site from the vial.

## Patentansprüche

1. Injektionsstelle (800), die mit einer stumpfen Kanüle verendbar ist und folgendes aufweist: ein Gehäuse (812), das einen durchgehenden Fluiddurchflußkanal definiert, eine elastische Dichtungseinheit, die von dem Gehäuse (812) über dem Kanal liegend getragen wird und ein erstes Ende des Kanals abdichtet, wobei die Dichtungseinheit mit einer wieder abdichtbaren Öffnung darin ausgebildet ist, die sich wenigstens zum Teil durch die Dichtungseinheit erstreckt und eine äußere Oberfläche hat; eine Festlegeeinrichtung zum Festlegen der Dichtungseinheit in dem Gehäuse derart, daß eine stumpfe Kanüle abdichtend durch die Öffnung eingeführt und in Fluiddurchflußverbindung mit dem Durchflußkanal gebracht werden kann, und derart, daß die Kanüle daraus entfernt werden kann, wobei die Dichtungseinheit mit der Festlegeeinrichtung so zusammenwirkt, daß die Öffnung wieder abgedichtet wird, wobei die Festlegeeinrichtung folgendes umfaßt: eine Verformung des ersten Endes des Gehäuses in Anlage an der äußeren Oberfläche der Dichtungseinheit, wobei diese Verformung des ersten Endes axial gerichtete Kräfte auf die Dichtungseinheit aufbringt, und eine Kupplungskomponente (814), die mit dem zweiten Ende integral ist und den Fluiddurchflußkanal weiterführt, wobei die Kupplungskomponente einen Dorn hat, der einen allgemein zylindrischen, hohlen Körperbereich (816) besitzt;
dadurch gekennzeichnet, daß der Körperbereich (816) des Dorns sich zu einem festen Mittelpunkt hin verjüngt, so daß der Stöpsel eines Arznei-Glasfläschchens durchstechbar ist, wobei der feste Mittelpunkt eine Öffnung hat, um das Ablaufen von Flüssigkeit aus dem Glasfläschchen durch den Fluiddurchflußkanal zu ermöglichen, wobei der Körperbereich (816) ein Widerhakenelement (818)umfaßt, um Widerstand gegen ein Lösen der Injektionsstelle von dem Glasfläschchen zu bieten.

## Revendications

1. Site d'injection (800) utilisable avec une canule épointée et comprenant :
un corps (812) définissant un canal traversant d'écoulement de fluide,
des moyens élastiques d'obturation portés par ledit corps (812) en recouvrement dudit canal, pour fermer une première extrémité du canal, les moyens d'obturation comportant un orifice refermable qui s'étend au moins en partie à travers lesdits moyens, et ayant une surface extérieure ;
des moyens de retenue pour retenir lesdits moyens d'obturation dans ledit corps de sorte qu'une canule épointée peut être insérée de façon étanche à travers ledit orifice et placée en communication d'écoulement de fluide avec ledit canal d'écoulement, et de sorte que la canule peut être retirée de cet orifice, lesdits moyens d'obturation coopérant avec lesdits moyens de retenue de manière à refermer ledit orifice, lesdits moyens de retenue comprenant une déformation de ladite première extrémité du corps contre ladite surface extérieure des dits moyens d'obturation, ladite déformation de première extrémité appliquant des forces axiales auxdits moyens d'obturation ; et
un composant de connexion (814) solidaire de la dite deuxième extrémité et en continuation dudit canal d'écoulement de fluide, ledit composant de connexion comprenant une pointe ayant un corps creux sensiblement cylindrique (816) ;
caractérisé en ce que le corps (816) de la pointe converge en une pointe centrale pleine pour permettre le perçage du bouchon d'un flacon de médicament, la pointe centrale pleine comportant une ouverture pour permettre le drainage de fluide à partir du flacon dans le canal d'écoulement de fluide, le corps (816) comportant une barbelure (818) pour créer une résistance contre la sortie du site d'injection hors du flacon.
